# EUROPEAN PATENT SPECIFICATION

(11) **EP 2 037 885 B1**
(45) Date of publication and mention of the grant of the patent: **21.01.2015**
(21) Application number: 07730010.1
(22) Date of filing: 08.06.2007
(51) Int. Cl.: A61K 9/16, A61K 9/20, A61K 31/4468, A61K 31/485, A61P 25/04

(54) **LOW DOSE SUBLINGUAL TABLETS OF OPIOID ANALGESICS AND PREPARATION PROCESS**
NIEDRIGDOSIERTE SUBLINGUALE TABLETTEN VON OPIOID-ANALGETIKA UND HERSTELLUNGSVERFAHREN
COMPRIMES FAIBLEMENT DOSES D'ANALGESIQUES OPIOIDES ET LEUR PROCEDE DE PREPARATION

(30) Priority: 09.06.2006 US 423339
(43) Date of publication of application: 25.03.2009
(73) Proprietor: ETHYPHARM, 92213 St Cloud Cedex (FR)
(72) Inventor: HERRY, Catherine, 76320 Saint Pierre Les Elbeuf (FR); OURY, Pascal, 78150 Le Chesnay (FR)
(74) Representative: Cabinet Plasseraud
(86) International application number: PCT/EP2007/055652
(87) International publication number: WO 2007/141328

(56) References cited:
- EP-A- 1 627 631
- EP-A1- 0 553 392
- WO-A-00/16750
- WO-A-99/40918
- WO-A-03/013479
- FR-A1- 2 771 291
- US-A- 5 202 128
- US-A- 5 411 745

## Description

The present invention relates to pharmaceutical tablets comprising low doses of an opioid analgesic as well a as to a method for preparing the same. The tablets of the invention are particularly useful for sublingual administration of said active ingredients.

The present invention further relates to a method of treating pain employing the tablets of the invention.

### BACKGROUND OF THE INVENTION

Sublingual administration has an advantage for active ingredients which, when given orally, are subject to a substantial first pass metabolism and enzymatic degradation through the liver, resulting in rapid metabolization and a loss of therapeutic activity related to the activity of the liver enzymes that convert the molecule into inactive metabolites, or the activity of which is decreased because of this bioconversion.

Sublingual route is capable of producing a rapid onset of action due to the considerable permeability and vascularization of the buccal mucosa. In the case of oral administration, the tablet is swallowed and systemic drug delivery occurs only at the level of the gastrointestinal mucosa, i.e. later.

Moreover, sublingual administration can also allow the administration of active ingredients which are not normally absorbed at the level of the stomach mucosa or digestive mucosa after oral administration, or alternatively which are partially or completely degraded in acidic medium after ingestion of the tablet.

An alternative to sublingual administration is transmucosal administration through the mucosa of the cheek. Fentanyl citrate, an opioid analgesic, is presently available in the form of a candy-on-a-handle (lollipop) for transmucosal administration which is marketed under the trade name Actiqo. The problem linked to this specific form is that the patient must keep the lollipop in the mouth for at least 15 minutes in order to obtain the desired amount of fentanyl. Furthermore, the amount of absorbed fentanyl is dependent of the frequency of saliva swallowing and thus very dependent of the patient. It is thus difficult to precisely check the absorbed amount of fentanyl. And finally, absorption through the mucosa of the cheek is generally less efficient than sublingual absorption.

For this reason, it is thought that the formulation of opioid analgesics, such as fentanyl, in the form of sublingual tablets would be advantageous.

The sublingual tablets known from the prior art are usually prepared by direct compression of a mixture of powders comprising the active ingredient and excipients for compression, such as diluents, binders, disintegrating agents and adjuvants.

In an alternative method of preparation, the active ingredient and the compression excipients can be dry- or wet-granulated beforehand.

In this case, the active ingredient is distributed throughout the mass of the tablet.

US 5 411 745 discloses morphine sulphate granules obtained by powder-layering a homogeneous mixture of morphine sulfate and hydrous impalpable onto inert beads.

WO 99/40918 discloses immediate release morphine sulfate microgranules comprising a neutral core coated with an active layer comprising sulphate morphine and a binder.

EP 1 627 631 discloses sustained release sulphate morphine granules comprising a neutral core coated with an active layer comprising sulphate morphine and a binder, and a sustained release layer.

FR 2 771 291 disclose spheroids which may be directly compressed with less than 5 % by weight of a lubricant. The disintegrating time of the tablet of example 2 is 45 minutes.

EP 0 553 392 discloses stabilized controlled release formulations having an acrylic polymer coating that increases storage stability.

WO 03/013479 discloses an oral dosage form of an opioïd analgesic to prevent abuse of opioids.

US 5 202 128 discloses a pellet composition providing a slow release of the active ingredient at a highly acidic pH and a constant relatively faster rate of release at a more alkaline pH. But no sublingual tablet is disclosed.

WO 00/16750 describes a tablet for sublingual use that disintegrates rapidly and comprises an ordered mixture in which the active ingredient is in the form of microparticles which adhere to the surface of watersoluble particles that are substantially greater in size, constituting a support for the active microparticles, the composition also comprising a mucoadhesive agent.

WO 00/57858 describes a tablet for sublingual use, comprising an active ingredient combined with an effervescent system intended to promote absorption, and also a pH-modifier.

Sublingual administration is an administration route which has certain limits due to the size of the sublingual cavity in which the tablet is placed, to the limited volume of saliva for solubilizing the active ingredient or else to the limited amount of active ingredient that can cross the buccal mucosa.

Because of these limits, tablets in which the active ingredient is distributed uniformly within the mass of the tablet have certain drawbacks that the present invention aims to solve.

A first drawback of these tablets in which the active ingredient is dispersed within the mass is the dependency which exists between the size of the tablet and the dosage of the active ingredient. Thus, if it is intended to provide tablets of various dosages, it will be necessary to have tablets of various sizes.

It may therefore be that the size of the tablet containing the highest dose, in particular its diameter, is no longer suitable for sublingual administration.

This may force those skilled in the art to modify the formula of the tablet containing the highest dose, in particular so as to adapt its size to sublingual use, which means, in the end, having tablets with different qualitative and/or quantitative formulae for one and the same active ingredient, which is neither economically desirable, nor desirable in terms of safety.

Moreover, sublingual administration requires the use of an active ingredient of specific particle size, usually consisting of a population for which the diameter is less than 10 µm, preferably less than 5 µm, as measured by the usual techniques, for example by laser diffraction.

This choice is aimed at ensuring rapid and complete solubilization of said active ingredient in the saliva and allowing immediate and sufficient systemic passage so as to obtain an instantaneous effect, which is highly desirable for the management of pain.

Now, the use of particles of this size in tablets means that it is also required to adapt the particle size of the excipients constituting the mass of the tablet and to very precisely define the mixing parameters for the pulverulent mass, in order to obtain an ordered mixture in which the active ingredient is uniformly distributed, without witnessing the appearance of a segregation phenomenon in the feed hopper of the tablet press, which would be liable to compromise the uniformity of content of the tablets during the compression.

The risk of appearance of a segregation phenomenon is further increased when the unit dose of active ingredient in each tablet is low. This is, for example, the case with fentanyl, for which the unit dose is generally less than a milligram to a few milligrams.

It is then difficult to obtain an acceptable uniformity of content for the same batch throughout the compression step, the active ingredient then being highly diluted in the pulverulent mixture of excipients.

For tablets for sublingual administration in which the active ingredient is uniformly dispersed in the mass, the release of the active ingredient is also dependent upon the rate of disintegration of the tablet.

The prior art describes tablets that disintegrate rapidly, suitable for sublingual administration, in which the active ingredient is distributed within the mass of the tablet.

It is known that these tablets usually have a low hardness, often less than 40 N, and exhibit a friability that is too great, such that they must be handled with care.

In the case of a tablet having a greater hardness, the disintegration time is increased, such that the tablet erodes gradually while releasing the active ingredient from the external surface of the tablet to its heart.

It is therefore particularly advantageous to have a formulation for sublingual administration that can rapidly release the active ingredient and allow immediate absorption thereof, without this release being dependent upon the rate of disintegration or upon the hardness of the tablet.

When the tablet is intended to disintegrate rapidly and without chewing, the disintegration leads to the formation of a pulp or of a suspension that can be unintentionally swallowed.

The viscosity of the pulp or suspension, which is related to the use of disintegrating agents or of swelling agents intended to accelerate the disintegration, can cause a swallowing reflex.

Consequently, part of the active ingredient is swallowed before being absorbed through the buccal mucosa.

The absorption at the level of the buccal mucosa, on which the bioavailability of the active ingredient directly depends, is therefore dependent upon the nature of the excipients used in this type of rapid disintegration formulation.

### SUMMARY OF THE INVENTION

The Applicant has demonstrated that it is possible to remedy all these drawbacks by means of a sublingual tablet comprising a low dose of active ingredient formed from microgranules. The tablets of the invention are particularly useful for sublingual administration of said active ingredients.

### DETAILED DESCRIPTION OF THE INVENTION

The sublingual tablet according to the invention comprises a directly compressible diluent in the form of neutral cores, wherein the neutral cores are coated with at least one layer comprising a low dose of an active ingredient (also referred to as "active layer" hereafter), and wherein the active ingredient is an opioid analgesic suitable for sublingual administration.

In the context of the present invention, the term "neutral cores" is understood to mean essentially spherical granules comprising sucrose and starch. Neutral cores particularly valued in the context of the invention comprise less than 91.5% of sucrose.

The present invention advantageously employs spherical particles, guaranteeing good flowability and good homogeneity of the blend to be tableted.

The excellent rheological properties of the neutral cores make them good candidates as direct compression excipients. The flow time of the neutral cores under the conditions of the test described in the European Pharmacopoeia is much less than 10 seconds. This property makes possible very efficient feeding of the tablet presses. In addition, the neutral cores have a very low compaction volume.

The neutral cores have the advantage of constituting a direct compression excipient which does not generate dust.

Finally, the neutral cores have a dissolution time much less than 15 minutes.

In addition, the present invention makes it possible to avoid the problems of demixing generally observed in direct compression as all the particles to be tableted have the same size.

The neutral cores have a size of between 100 and 2000 µm, preferably between 150 and 600 µm, or preferably between 150 and 500 µm, e.g. 180 to 250 µm or 400 to 500 µm.

Active ingredients that are advantageous in the tablet according to the invention are the active ingredients that are suitable for buccal or sublingual administration due to their pharmacokinetic characteristics, in particular when the active ingredient has a window of absorption in the buccal cavity, or a considerable first-pass affect through the liver requiring an alternative route of administration to conventional oral administration, or alternatively when it is sought to obtain a very rapid systemic effect to overcome the effects of an attack such as an attack of angina pectoris, an anxiety attack, acute pain, an allergic attack, an asthma attack, or a withdrawal attack caused by, for example, opiate or alcohol withdrawal.

Examples of such active ingredients are provided in the publication "Oral Mucosal Drug Delivery", Hao Zhang et al., Clin. Pharmacokinet. 2002, 41, (9) 661-680.

In the present invention the active ingredient is chosen from opioid analgesics suitable for sublingual administration. Examples of suitable opioid analgesics comprise buprenorphine, nor-buprenorphine, fentanyl, methadone, levorphanol, morphine, hydromorphone, oxymorphone codeine, oxycodone, hydrocodone and their pharmaceutically acceptable salts.

In the present invention, "fentanyl" as active ingredient, is intended to mean fentanyl and derivatives thereof.

Derivatives of fentanyl comprise alfentanil, sufentanil and remifentanil.

The term "pharmaceutically acceptable salts" is intended to mean the derivatives of the compounds described in which the pharmaceutically active base compound is converted to its salt with a base or acid, examples of pharmaceutically active salts comprising in particular organic and inorganic acid salts of basic residues, such as amines, alkali metal derivatives or organic salts of acidic residues, such as carboxylic acids, and the like.

Examples of pharmaceutically acceptable salts of fentanyl comprise fentanyl citrate and fentanyl hydrochloride. Examples of pharmaceutically acceptable salts of derivatives of fentanyl comprise alfentanil hydrochloride, sufentanil citrate and remifentanil hydrochloride.

Preferred active ingredients are fentanyl base, fentanyl citrate, alfentanil, alfentanil hydrochloride, sufentanil, sufentanil citrate, remifentanil, remifentanil hydrochloride.

Active ingredients may be used in any polymorphic form, in racemic form or in form of their enantiomers or in form of mixture of enantiomers.

The active ingredient can be in the form of a powder, for example a micronized powder or of microcrystals.

The amount of active ingredient is preferably less than 20 mg/tablet, more preferably less than 10 mg/tablet, eg. 0.1 to 10 mg/tablet, preferably 0.1 to 2 mg/tablet. The amount of active ingredient is adjusted within the above limits according to the type of active ingredient.

Given that the tablets are low dose ones, it is usually not necessary to add further excipients to the coating composition comprising the active ingredient applied over the neutral cores (also referred hereunder as the "active layer"). The microgranules are preferably composed of a neutral core, at the surface of which the active ingredient is adsorbed.

In a preferred embodiment the active layer is devoid of any excipient.

If, despite everything, excipients prove to be preferable in carrying out the application of the active layer on the neutral cores, the choice of their respective composition and amount will be such that they do not substantially modify the tableting properties of the neutral cores or modify the release of the active ingredient.

The pharmaceutically acceptable excipients optionally present in the active layer are chosen from binders, soluble agents, surfactants, absorption promoters, bioadhesive agents, antistatic agents, acid/base pairs that produce an effervescence, sweeteners, flavorings, colorants, and mixtures thereof.

The binder, which is optionally present in the active layer, is used in proportions that can range up to 95% by weight relative to the dry weight of the coating, preferably up to 30% by weight relative to the dry weight of the active layer.

Its role is to bind the active ingredient to the neutral core without loss of material, or to form a homogeneous layer of active ingredient and other excipients, evenly distributed around the neutral core.

The binder is preferably chosen from polymers that are hydrophilic and/or soluble at the pH of saliva, so as to allow an instant release of the active ingredient, such as polyvinylpyrrolidones and cellulose-based polymers, acrylic polymers and polyethylene glycols.

The polyvinylpyrrolidone can be chosen from polymers having a molecular mass of between 10 000 and 50 000.

The cellulose-based polymer is chosen from hydroxylated derivatives, for example hydroxypropylmethylcellulose, hydroxypropylcellulose, hydroxymethylcellulose, hydroxypropylmethylcellulose phthalate and hydroxypropylmethylcellulose acetosuccinate.

The preferred hydroxypropylmethylcellulose is chosen from those for which the apparent viscosity (aqueous solution at 2% m/m, at 20°C, USP method) is between 2.4 and 18 cP, and even more preferably between 2.4 and 5 cP.

The preferred polyethylene glycol is chosen from those for which the nominal molecular mass is 4000 or 6000 g/mol.

The soluble agent, which may be optionally present in the active layer, is used in a proportion that can range up to 90% by weight, preferably of between 1% and 60%, and even more preferably of between 30 and 60% by weight, calculated relative to the dry weight of the active layer.

This soluble agent is used in particular for improving the solubilization of the active ingredient by accelerating the solubilization of the coating comprising the active ingredient.

The soluble agent can be chosen from the group of sugars such as sucrose, lactose or dextrose, of polyols such as mannitol, sorbitol or lactitol, or else of inorganic salts such as sodium chloride.

The surfactant, which is optionally present in the active layer, can be chosen from cationic, anionic, nonionic or amphoteric agents, alone or as a mixture. The surfactant can be chosen, for example, from compounds such as sodium lauryl sulphate, the monooleate, the monolaurate, the monopalmitate, the monostearate, the trioleate, the tristearate or any other ester of polyoxyethylenated sorbitan, preferably Tween® 20, 40, 60 or 80, glycerides of polyoxyethylenated fatty acids, these fatty acids being saturated or unsaturated and composed of at least 8 carbon atoms, poloxamers, such as poloxamer 188, ethylene oxide/propylene oxide block copolymers, such as Pluronic® F68 or F87, lecithin, stearyl alcohol, cetearyl alcohol, cholesterol, polyoxyethylenated castor oil, fatty alcohol polyoxyethylenated ethers, such as the Brij® products, and polyoxyethylenated stearates.

The surfactant is advantageously present in a proportion that can range up to 20%, preferably of between 0.1 and 20% by weight relative to the total dry weight of the active layer.

The absorption promoters, which are optionally present in the active layer, are compounds that make it possible to improve the absorption of the active ingredient through the walls of the buccal cavity to the bloodstream.

These compounds can be chosen from the group comprising, for example, sodium lauryl sulphate, sodium caprate or chitosans, and also P-glycoprotein (P-gp) inhibitors, such as polysorbate 80, Cremophor® EL (hydrogenated castor oil) or Solutol® HS-15 (PEG-HS or polyethylene glycol-660 12-hydroxystearate).

The bioadhesive agents, which are optionally present in the active layer, can be chosen from the group comprising, for example, carbomers, sodium carboxymethylcellulose, sodium alginate, hydroxypropylmethylcellulose, hydroxypropylcellulose, hydroxyethylcellulose, ethylcellulose, gelatine, guar gum, poly(ethylene oxide)s (trade name Polyox®) and dextran.

The antistatic agent, which is optionally present in the active layer, can be chosen from the group consisting of colloidal silica and preferably precipitated silica, micronized talc and mixtures thereof.

The antistatic agent is used in a proportion that can range up to 60% by weight, calculated relative to the dry weight of the coating applied around the compressed core.

The acid/base pair that produces an effervescence, which is optionally present in the active layer, is formed from an alkaline agent and an acidic agent which are chosen from those that are pharmaceutically acceptable, such that, in the presence of water, they allow the release of a gas.

The advantage of using an effervescent mixture in the coating comprising the active ingredient is that of facilitating the rapid dissolution of the active layer formed around the microgranules upon contact with saliva, and thus obtaining, through the release of a pharmaceutically acceptable gas and induction of a buccal micro pH, rapid solubilization of the active ingredient at the buccal or sublingual mucous membranes and an improved systemic drug delivery while at the same time improving the organoleptic properties so as to decrease the feeling of the active ingredient in the buccal cavity, or inducing a pleasant slightly acid taste.

The acidic agent is a proton-donating compound which can react with an alkaline agent so as to form a gas which causes the effervescence of the liquid in which this gas is released.

The acidic agent can consist of any inorganic or organic acid, in the form of a free acid, an acid anhydride or an acid salt.

This acid is chosen from the group comprising in particular tartaric acid, citric acid, maleic acid, fumaric acid, malic acid, adipic acid, succinic acid, lactic acid, glycolic acid, alpha-hydroxy acids, ascorbic acid and amino acids, and also the salts and derivatives of these acids.

The alkaline agent consists of a compound capable of generating a gas by reaction with a proton-donating compound. The gas formed is carbon dioxide, oxygen or any other type of biocompatible gas.

The alkaline agent is chosen from the group comprising potassium carbonate, lithium carbonate, sodium carbonate, calcium carbonate, ammonium carbonate, L-lysine carbonate, arginine carbonate, sodium glycine carbonate, sodium carbonates of amino acids, anhydrous sodium perborate, effervescent perborate, sodium perborate monohydrate, sodium percarbonate, sodium dichloroisocyanurate, sodium hypochlorite, calcium hypochlorite, and mixtures thereof.

In the context of the present invention, the term "carbonate" is intended to mean, without distinction, carbonates, sesquicarbonates and hydrogen carbonates.

The respective amounts of acidic agent and of alkaline agent are adjusted such that the reaction between the alkaline agent and the protons released by the acid allows the generation of a sufficient amount of gas to obtain a satisfactory effervescence.

Acidic agent alone or alkaline agent alone can be used in adapted quantity in the coating to ensure that the active ingredient will be mainly under base form, i.e. absorbable fraction.

Suitable sweeteners, which are optionally present in the active layer, may be selected from the group comprising in particular aspartame, acesulfam potassium, sodium saccharinate, neohesperidine dihydrochalcone, sucralose, monoammonium glycyrrhizinate, and mixtures thereof.

Suitable flavorings and colorants, which are optionally present in the active layer, are those commonly used in pharmacy for the preparation of tablets.

The incorporation of sweetener(s) and/or flavorings in the coating of the neutral cores of the invention is particularly advantageous in order to mask the bitterness of certain active ingredients.

The colorants are those normally used in pharmacy. The colorant is used in a proportion that can range up to 1% by weight, calculated relative to the dry weight of the layer applied around the neutral core.

In a particular embodiment, the active layer does neither comprise sweeteners nor flavourings.

The composition of excipients within the active layer is adjusted such that the active ingredient completely solubilizes when the tablet disintegrates.

The neutral cores comprising an active layer (defined hereunder as "active core(s)") may optionally be coated with a layer comprising a pH-modifying compound, said layer being named pH-modifying layer.

According to one embodiment, the pH-modifying layer can be present above or under the active layer.

According to another embodiment, the pH-modifying compound can be present within the active layer.

The pH-modifying layer allows the provision of a local alkaline or acid pH when the tablet is placed in the buccal cavity which enhances the absorption of active ingredient by the mucosa.

Suitable pH-modifying compounds comprise citric acid and sodium citrate or potassium citrate, sodium hydroxide, monoethanolamine, diethanolamine, sodium bicarbonate or potassium bicarbonate, sodium phosphate, tartaric acid, propionic acid, lactic acid, malic acid and monosodium glutamate.

The choice of the pH-modifying compound depends on the nature of the active ingredient used. In the case where absorption of the active ingredient by the buccal mucosa is enhanced under alkaline conditions, an alkaline compound will be used as pH-modifying compound. In the case where absorption of the active ingredient by the buccal mucosa is enhanced under acid conditions, an acidic compound will be used as pH-modifying compound.

The composition of the pH-modifying layer is adjusted such that when the active ingredient completely solubilizes upon contact with the saliva, the pH around the buccal area is adjusted at a value favorable for the sublingual absorption of the active ingredient.

The pH-modifying layer may optionally comprise excipients identical to those present in the active layer.

In the case of fentanyl as active ingredient, the optional pH-modifying layer is an alkaline layer comprising an alkaline compound.

According to one embodiment, the alkaline coating layer may be present above or under the fentanyl layer.

According to another embodiment, the alkaline compound can be present within the fentanyl layer.

Said alkaline layer allows the provision of a local alkaline pH when the tablet is placed in the buccal cavity which enhances the absorption of fentanyl through the mucosa.

The alkaline layer may optionally comprise excipients identical to those present in the layer comprising fentanyl.

The alkaline compound is advantageously selected from the group comprising tris, tartrate, acetate, phosphate, and preferably anhydrous disodium phosphate and mixtures thereof.

The invention also relates to a method of preparing the neutral cores of the tablet of the invention.

Said method comprises applying an active layer to the neutral cores by spraying a solution, suspension or colloidal dispersion comprising an active ingredient suitable for sublingual administration and, optionally, at least one pharmaceutically acceptable excipient, onto the neutral cores.

Active ingredients and excipients are those described above in relation to the tablet of the invention.

In a preferred embodiment, the active ingredient is fentanyl or a pharmaceutically acceptable salt thereof, in any polymorphic form, in racemic or enantiomeric form.

The active layer is distributed uniformly over the surface of the neutral microgranules.

In particular, for water-insoluble substances, it is possible to form a layer in which the active ingredient is in the form of a solid dispersion, obtained by coprecipitation of the active ingredient with a hydrophilic polymer.

Spraying can be carried out in a perforated drum or in a fluid bed coater. In a preferred embodiment, the spraying of the active layer onto the neutral microgranules is carried out in a perforated drum, in particular in a perforated drum having sections with triangular profiles, parallel to one another and defining the apertures between them, such as that described in patent application EP 1044064.

The coating composition is sprayed in the form of a solution, a suspension or a colloidal dispersion in an organic or aqueous solvent, or mixtures thereof, and is then dried.

The organic solvent can be chosen from ethanol, isopropanol, tetrahydrofuran, isopropyl ether, acetone, methyl ethyl ketone, methylene chloride or a mixture of these solvents.

Purified water is the solvent preferably used if the coating is devoid of effervescent agents; on the other hand, an organic solvent has to be used when the sprayed composition contains an effervescent acid/base pair.

In one embodiment, the method of preparing the neutral cores comprises a step of applying a pH-modifying layer to said cores, preferably by spraying a solution, suspension or colloidal dispersion comprising a pH-modifying compound and, optionally, at least one pharmaceutically acceptable excipient, onto the neutral cores.

In the case where the active ingredient is fentanyl or a pharmaceutically acceptable salt thereof, the pH-modifying compound is an alkaline compound and the pH-modifying layer an alkaline layer.

The pH-modifying layer may be applied simultaneously with the active layer or above or under the active layer.

Spraying of the pH-modifying layer can be carried out for example in a perforated drum or in a fluid bed coater.

In a preferred embodiment, the spraying of the pH-modifying layer onto the neutral cores is carried out in a perforated drum, in particular in a perforated drum having sections with triangular profiles, parallel to one another and defining the apertures between them, such as that described in patent application EP 1044064.

The choice of the equipment makes it possible to control the application of the pH-modifying layer and to prevent any sticking phenomena, linked to the nature of the active ingredient and of the excipients of the pH-modifying coating composition, and to the various parameters of the method (temperature, air pressure, solution flow rate).

The coating composition containing the pH-modifying compound is sprayed in the form of a solution, a suspension or a colloidal dispersion in an organic or aqueous solvent, or mixtures thereof, and is then dried.

The amount of binder is adjusted according to the nature and the amount of active ingredient sprayed over neutral cores.

In particular, for water-insoluble substances, it is possible to form a layer in which the active ingredient is in the form of a solid dispersion, obtained by coprecipitation of the active ingredient with a hydrophilic polymer.

The solvent used for the application of the coating will generally be water or any other authorized solvent with an appropriate drying stage.

Purified water is the solvent preferably used if the coating is devoid of effervescent agents; on the other hand, an organic solvent has to be used when the sprayed composition contains an effervescent acid/base pair.

The organic solvent can be chosen from ethanol, isopropanol, tetrahydrofuran, isopropyl ether, acetone, methyl ethyl ketone, methylene chloride or a mixture of these solvents.

The sublingual tablet of the invention can be prepared by means of a method comprising at least the following steps:
1. Preparation of microgranules comprising a layer comprising an active ingredient by spraying a solution or a suspension comprising an active ingredient suitable for sublingual administration and, optionally, at least one pharmaceutically acceptable excipient, onto the neutral cores; and
2. Compression of the microgranules obtained in step 1 so as to obtain a tablet.

In one embodiment, step 1 of the method of preparing the tablets of the invention comprises a step of applying a pH-modifying layer to said cores.

The pH-modifying layer may be applied simultaneously with the active layer or above or under the active layer.

The method of the invention is advantageous in terms of safety since it avoids the handling of active ingredients in the form of pulverulent mixtures, as is the case in conventional granulation and/or compression steps, and allows the product to be contained by using the active ingredient in the form of a sprayed solution or suspension.

Especially in the case of highly toxic active ingredients, it will be appreciated that the method of the invention avoids the handling of these substances in the form of pulverulent mixtures, as is the case in the traditional granulation and/or compression steps, and allows the highly toxic active ingredient to be contained by using the active ingredient in the form of a sprayed solution or suspension.

The conditions and details of step 1 of the method of preparing the tablets of the invention are as described above in respect of the method of preparing the neutral cores of the invention.

Optionally, one or more lubricant can be added in the compression step to the microgranules obtained in step 1.

Said lubricant(s) can be present in an amount of less than 1% by weight with respect to the weight of the tablet, preferably of between 0.10 and 0.75% by weight, more preferably of the order of 0.10% to 0.50% by weight, of the tablet.

The lubricant makes it possible to reduce friction between particles and between particles and the press mold. It also makes it possible to reduce adhesion of the particles to the punches and to obtain a degree of gloss. The lubricant is chosen, for example, from magnesium, zinc or calcium stearate, talc, Aerosil®, stearic acid, sodium stearylfumarate and PEGs.

The sublingual tablets of the present invention exhibit a uniformity in mass of much less than 5% and of the order of 1% for tablets with a mass of the order of 300 to 500 mg, a friability of less than 1%, a dissolution time at 37°C of less than 15 minutes, and a hardness of the order of 0 to 200 N. These parameters can be adjusted by the interplay of the tableting parameters.

The compression force applied in step 2 is advantageously between 5 and 50 kN when the compression surface area is 1 cm² (i.e. 50 to 500 MPa), preferably between 10 and 30 kN. The compression force is adjusted so as to obtain a tablet which hardness is preferably between 10 and 180 N, more preferably between 15 and 100 N, measured according to the method of the European Pharmacopoeia (2.9.8).

Preferably, the hardness of the tablet of the invention is adjusted so as to obtain a friability, measured according to the method of the European Pharmacopoeia, of less than 1% by weight.

An advantage of the tablets according to the invention is that they have a disintegration time of less than 15 min, preferably of 5 to 15 min.

The disintegration time is measured in vivo by placing the coated tablet in the sublingual cavity, and measuring, using a stopwatch, the time that elapses between the beginning of the measurement and the moment when the coated tablet has completely disintegrated under the action of saliva and without chewing, so as to form only a viscous pulp, the patient not having to use, during all this time, any action of the jaws.

The tablets of the invention can have a diameter of between 2 and 14 mm and a round, oval, oblong or other shape, can have a flat, convex, or other surface, and can optionally have engraving.

Preferably, the tablets of the invention have a round biconvex shape, which is an advantageous shape for both the tableting process and the contact of the coated tablet with saliva when said tablet is placed in the buccal cavity.

According to a particular embodiment, the sublingual tablets according to the invention can be film-coated, either to improve their appearance or to mask the color or to protect the active ingredient from light, moisture or oxygen in the air.

According to an another embodiment of the invention, it is possible to use coloring agents in the coating of the tablet as a code to indicate the type and dosage of active ingredient. In fact, whatever the dosage is, the size of the tablets can be the same. In order to make the difference between different dosages, a specific color can be associated to a specific dosage.

In a particularly advantageous embodiment, the method for preparing a sublingual tablet according to the invention, comprises at least the following steps:
1. Preparation of microgranules comprising an opioid analgesic suitable for sublingual administration, preferably fentanyl as active ingredient and, optionally pharmaceutically acceptable excipients, onto the neutral cores; and
2. Compression of the microgranules obtained in step 1, optionally with a lubricant, so as to obtain a tablet;
wherein the term "fentanyl" is to be understood as defined above.

The conditions and details of steps 1 and 2 of this embodiment are as described above in respect of the general method for preparing tablets according to the invention.

Optionally, the method of preparing the tablets of the invention comprises spraying a solution or suspension also comprising an alkaline compound onto the neutral cores. Said step can be carried out directly onto the neutral cores (before step 1), or simultaneously with the spraying step of the coating containing the active ingredient (simultaneously with step 1), or onto the active core obtained from step 1.

According to a particular option, the fentanyl-containing sublingual tablets according to the invention can be film-coated, either to improve their appearance or to mask the color or to protect the active ingredient from light, moisture or oxygen in the air.

The present invention is also directed to a tableting premix which consists of a composition containing 99 to 100% by weight of neutral cores coated with at least one active layer comprising a low dose of an opioid analgesic suitable for sublingual administration and 0 to 1% by weight of a lubricant. Said composition is intended to be subjected to direct compression.

The coated cores of the tableting premix according to the invention may comprise a pH-modifying layer as described here above in relation to the neutral cores comprising an active layer and their preparation.

The active ingredient preferably represents less than 5% by weight of the neutral cores.

In a preferred embodiment, the opioid analgesic is fentanyl, wherein the term "fentanyl" is to be understood as defined above.

The present invention is also directed to a process for the preparation of the sublingual tablets of the invention by direct compression of the above tableting premix. According to this process, the compression force is advantageously between 5 and 50 kN when the compression surface area is 1 cm² (i.e. 50 to 500 MPa), preferably between 10 and 30 kN.

Furthermore, the present invention relates to a method of treating pain which comprises introducing into the buccal cavity of a patient a therapeutically effective amount of an active ingredient in the form of a sublingual tablet wherein the active ingredient is selected from the group comprising opioid analgesics suitable for sublingual administration, such as buprenorphine, nor-buprenorphine, fentanyl, alfentanil, sufentanil, remifentanil, methadone, levorphanol, morphine, hydromorphone, oxymorphone codeine, oxycodone, hydrocodone and their pharmaceutically acceptable salts.

Particularly preferred active ingredients are fentanyl, fentanyl citrate, alfentanil, alfentanil hydrochloride, sufentanil, sufentanil citrate, remifentanil, remifentanil hydrochloride.

In a particularly advantageous embodiment, the method of treating pain according to the present invention comprises introducing into the buccal cavity of a patient a therapeutically effective amount of a sublingual tablet comprising a directly compressible diluent in the form of neutral cores, wherein the neutral cores are coated with a low dose of fentanyl as active ingredient, wherein the term "fentanyl" is to be understood as defined above.

The method of treating pain according to the present invention is particularly useful for treating breakthrough pain, in particular breakthrough cancer pain. It is particularly suitable for treating patients who are already receiving and who are tolerant to opioid therapy for their underlying persistent pain.

Patients considered opioid tolerant are those who are taking at least 60 mg morphine/day, at least 25 µg transdermal fentanyl/hour, at least 30 mg of oxycodone daily, at least 8 mg of oral hydromorphone daily or an equianalgesic dose of another opioid for a week or longer.

The invention also relates to the use of an opioid analgesic suitable for sublingual administration, such as buprenorphine, nor-buprenorphine, fentanyl, alfentanil, sufentanil, remifentanil, methadone, levorphanol, morphine, hydromorphone, oxymorphone codeine, oxycodone, hydrocodone and their pharmaceutically acceptable salts, for the manufacture of a sublingual tablet according to the invention.

Fentanyl and its derivatives in any polymorphic form, in racemic form or in the form of their enantiomers or in the form of mixtures of enantiomers, in the base form or in the form of a pharmaceutically acceptable salt are preferred active principles. Particularly preferred active principles are fentanyl, fentanyl citrate, alfentanil, alfentanil hydrochloride, sufentanil, sufentanil citrate, remifentanil, remifentanil hydrochloride.

The so obtained sublingual tablets according to the invention are particularly useful for treating breakthrough pain, in particular breakthrough cancer pain. It is particularly suitable for treating patients who are already receiving and who are tolerant to opioid therapy for their underlying persistent pain.

The invention will be understood more clearly from the following example, without the latter in any way limiting the scope of said invention.

### EXAMPLES

In the following examples, the below-mentioned products are used:
Neutral cores "Neutres SP" (400 - 500 µm) available from NPPHARM.
Neutral cores "NPTAB 200" (180 - 250 µm) available from NPPHARM.
HPMC 603 (hydroxypropylmethylcellulose) available under the trademark Pharmacoat 603 from Shin-Etsu.
PEG 6000 available under the trademark Renex PEG 6000 from Quimasso.
Magnesium stearate available from Quimdis.

The given percentages are expressed by weight.

### Example 1

### 1 - Coating of the neutral cores

The coating solution of fentanyl citrate and PEG 6000 in water is sprayed, in a fluid bed coater, onto 1000 g of neutral cores SP.

The formula of the coated microgranules is given in table 1.

**Table 1**

| **Material** | **Batch Formula (g)** | **% Formula** |
|---|---|---|
| Neutral cores SP | 1000.0 | 98.54 |
| Fentanyl citrate | 1.3 | 0.13 |
| PEG 6000 | 13.5 | 1.33 |
| Water | 167.5 | - |
| Total (dry weight) | 1014.8 | 100 |

### 2 - Lubrication and compression

The coated microgranules are lubricated with 0.12% of magnesium stearate.

The microgranules are then compressed on an alternating press (Frogerais OA) equipped with round flat chamfered punches, 11 mm in diameter.

The obtained tablets according to the invention have a unit dosage of 0.63 mg of fentanyl citrate, i.e. of 0.4 mg of fentanyl base.

### Example 2

### 1 - Coating of the neutral cores with fentanyl citrate

The coating solution of fentanyl citrate and HPMC 603 in water is sprayed, in a fluidized air bed, onto 700 g of neutral cores NPTAB 200.

The formula of the coated microgranules is given in table 1.

**Table 2**

| **Material** | **Batch Formula (g)** | **% Formula** |
|---|---|---|
| Neutral cores NPTAB 200 | 700.0 | 96.34 |
| Fentanyl citrate | 6.3 | 0.87 |
| HPMC 603 | 20.0 | 2.79 |
| Purified water | *581.0* | - |
| Total (dry weight) | 167.5 | 100 |

### 2 - Lubrication and compression

The coated microgranules are lubricated with 0.22% of magnesium stearate.

The microgranules are then compressed on an alternating press (SVIAC PR12) equipped with round concave punches, 5.5 mm in diameter.

The formula of the obtained tablets according to the invention is given in table 3.

**Table 3**

| Material | Batch Formula (g) | % Formula |
|---|---|---|
| NPTAB 200 coated with fentanyl citrate | 970.0 | 99.78 |
| Magnesium stearate | 2.17 | 0.22 |
| Total | 972.17 | 100 |

The obtained tablets according to the invention have a unit dosage of 0.63 mg of fentanyl citrate, i.e. of 0.4 mg of fentanyl base.

### Example 3

A Crossover Single-Dose Comparative Bioavailability Study of Tablets prepared according to example 2 versus Actiq® 0.4 mg was performed in Healthy Male Volunteers under Fasting Conditions

The objective of this pilot study was to assess the single-dose relative bioavailability of both formulations in healthy male volunteers under fasting conditions.

Tablets prepared according to example 2 and the reference product which were each administered to 10 patients and the Cmax, Tmax and AUC were measured.

The reference product is a fentanyl citrate formulation (solid drug matrix on a handle) designed to facilitate transmucosal absorption and marketed worldwide under trademark Actiq®.

Both the invention and the reference product contain fentanyl citrate in an amount equivalent to 0,4 mg of fentanyl base.

Blood sampling points: before dosing and at the following times thereafter in each period: 5, 10, 15, 20, 25, 30, 35, 40, 45, 50, 60, 75, 90 minutes and at 2, 3, 4, 6, 8, 12 and 24 hours post-dose

The AUC 0-t, AUC_{∝}, Cmax, tmax pharmacokinetic parameters were calculated for fentanyl in plasma.

Geometric means are given in Table 4:

**Table 4**

| **Parameter**(**CV%)** | **Invention (n=10)** | **Actiq^{®} 0,4 mg(n=10)** |
|---|---|---|
| **AUC 0-t (pg·h/mL)** | 3526.80 (22.5%) | 3059.25 (33.1%) |
| **AUC_{∝} (pg·h/mL)** | 4079.90 (28.8%) | 3473.98 (37.0%) |
| **Cmax (pg/mL)** | 491.67 (29.4%) | 406.614 (27.1%) |
| **tmax* (h)** | 2.5 [0.42 - 3.00] | 2.0 [1.0 - 3.0] |

| | | |
|---|---|---|
| *median [range] | | |

Mean Ratios of the invention versus Actiq® are calculated in table 5 below

**Table 5**

| Parameter | Invention vs. Actiq® |
|---|---|
| AUC 0-t | 110.7% |
| AUC_{∝} | 112.6% |
| Cmax | 113.5% |

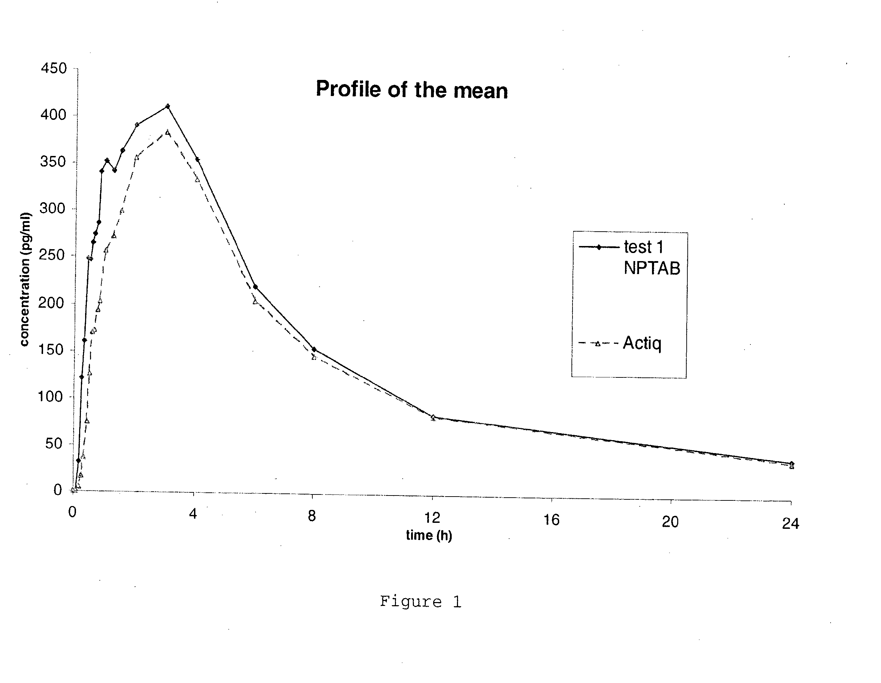

The results are also presented in Figure 1.

The Invention shows similar bioavailability and Cmax than Actiq®.

## Claims

1. A sublingual tablet comprising less than 20mg/tablet of an opioid analgesic suitable for sublingual administration, comprising a directly compressible diluent in the form of neutral cores, wherein said neutral cores are essentially spherical granules comprising sucrose and starch, and wherein said neutral cores are coated with at least one active layer comprising less than 5% by weight of the neutral core of said opioid analgesic suitable for sublingual administration.

2. The sublingual tablet as claimed in claim 1, wherein the opioid analgesic is a member selected from the group consisting of buprenorphine, nor-buprenorphine, fentanyl, methadone, levorphanol, morphine, hydromorphone, oxymorphone codeine, oxycodone, hydrocodone and their pharmaceutically acceptable salts, in any polymorphic form, in racemic or enantiomeric form.

3. The sublingual tablet as claimed in claim 1, wherein the opioid analgesic is a member selected from the group consisting of fentanyl base, fentanyl citrate, alfentanyl, alfentanyl hydrochloride, sufentanyl, sufentanil citrate, remifentanil and remifentanil hydrochloride.

4. The sublingual tablet according to any one of claims 1 to 3, wherein the active layer is devoid of any excipient.

5. The sublingual tablet according to any one of claims 1 to 4, wherein the neutral cores are coated with a pH modifying layer.

6. The sublingual tablet according to claim 3, wherein the neutral cores are coated with an alkaline layer.

7. The sublingual tablet according to any one of claims 1 to 6, wherein a size of the neutral cores is from 100 to 2000 µm, preferably from 200 to 600 µm, more preferably from 200 to 400 µm.

8. The sublingual tablet according to any one of claims 1 to 7, having a hardness between 0 and 200 N.

9. The sublingual tablet according to any one of claims 1 to 8, having friability between 0 and 1%.

10. The sublingual tablet according to any one of claims 1 to 9, having a disintegration time of less than 15 minutes.

11. The sublingual tablet according to any one of claims 1 to 10, wherein the tablet further comprises one or more lubricants in an amount of less than 1% by weight with respect to a weight of the tablet, preferably between 0.125 and 0.75% by weight with respect to a weight of the tablet, more preferably about 0.25% by weight with respect to a weight of the tablet.

12. The sublingual tablet according to any one of claims 1 to 11, wherein an amount of active ingredient is less than 5 mg/tablet.

13. A method for preparing a sublingual tablet according to any one of claims 1 to 12 comprising at least the following steps:
(1) preparation of microgranules comprising an opioid analgesic suitable for sublingual administration by spraying a solution, suspension or colloidal dispersion comprising said opioid suitable for sublingual administration onto neutral cores; and
(2) compression of the microgranules obtained in step 1 so as to obtain the sublingual tablet.

14. The method according to claim 13, wherein the solution, suspension or colloidal dispersion comprising said opioid analgesic suitable for sublingual administration is devoid of any excipient.

15. The method according to claim 13 or 14, wherein the solution, suspension or colloidal dispersion comprising the opioid analgesic further comprises at least one pharmaceutically acceptable excipient.

16. The method according to any one of claims 13 to 15, wherein the opioid analgesic is selected from the group consisting of buprenorphine, nor-buprenorphine, fentanyl, methadone, levorphanol, morphine, hydromorphone, oxymorphone codeine, oxycodone, hydrocodone and their pharmaceutically acceptable salts, in any polymorphic form, in racemic or enantiomeric form.

17. The method according to any one of claims 13 to 16, wherein the opioid analgesic is selected from the group consisting of fentanyl base, fentanyl citrate, alfentanyl, alfentanyl hydrochloride, sufentanyl, sufentanil citrate, remifentanil, remifentanil hydrochloride.

18. The method according to claim 17 wherein step 1 of the method comprises a step of applying an alkaline layer.

19. The method according to any one of claims 13 to 18, wherein step 1 of the method comprises a step of applying a pH-modifying layer.

20. The sublingual tablet according to anyone of claims 1 to 12, for use in treating pain, such as breakthrough pain, in particular breakthrough cancer pain.

21. The sublingual tablet according to claim 20, for use in treating pain in a patient who is already under opioid therapy.

22. Use of the tablet according to any one of claims 1 to 12 for the manufacture of a medicament for the treatment of pain by sublingual administration of said tablet.

23. Use according to claim 22, wherein said pain is breakthrough pain, in particular breakthrough cancer pain.

24. Use according to claim 23, for the treatment of pain in a patient who is already under opioid therapy.

## Patentansprüche

1. Sublinguale Tablette, welche weniger als 20 mg/Tablette eines opioiden Analgetikums, geeignet zur sublingualen Verabreichung, umfasst, welche ein direkt pressbares Verdünnungsmittel in Form neutraler Kerne umfasst, wobei die neutralen Kerne im Wesentlichen spherische Granulate sind, welche Saccharose und Stärke umfassen, und wobei die neutralen Kerne mit mindestens einer aktiven Schicht beschichtet sind, welche weniger als 5 Gewichts-% des neutralen Kerns des opioden Analgetikums, geeignet zur sublingualen Verabreichung, umfasst.

2. Sublinguale Tablette nach Anspruch 1, wobei das opioide Analgetikum ein Mitglied ist ausgewählt aus der Gruppe bestehend aus Buprenorphin, nor-Buprenorphin, Fentanyl, Methadon, Levorphanol, Morphin, Hydromorphon, Oxymorphon, Codein, Oxycodon, Hydrocodon und deren pharmazeutisch annehmbaren Salzen, in einer polymorphen Form, in einer racemischen oder enantiomeren Form.

3. Sublinguale Tablette nach Anspruch 1, wobei das opioide Analgetikum ein Mitglied ist ausgewählt aus der Gruppe bestehend aus Fentanylbase, Fentanylcitrat, Alfentanyl, Alfentanylhydrochlorid, Sufentanyl, Sufentanilcitrat, Remifentanil und Remifentanilhydrochlorid.

4. Sublinguale Tablette nach einem der Ansprüche 1 bis 3, wobei die aktive Schicht frei von Hilfsstoff ist.

5. Sublinguale Tablette nach einem der Ansprüche 1 bis 4, wobei die neutralen Kerne mit einer pH-modifizierenden Schicht beschichtet sind.

6. Sublinguale Tablette nach Anspruch 3, wobei die neutralen Kerne mit einer alkalischen Schicht beschichtet sind.

7. Sublinguale Tablette nach einem der Ansprüche 1 bis 6, wobei eine Größe der neutralen Kerne von 100 bis 2000 µm beträgt, bevorzugt von 200 bis 600 µm, stärker bevorzugt von 200 bis 400 µm.

8. Sublinguale Tablette nach einem der Ansprüche 1 bis 7, welche eine Härte zwischen 0 und 200 N aufweist.

9. Sublinguale Tablette nach einem der Ansprüche 1 bis 8, welche Zerreibbarkeit zwischen 0 und 1% aufweist.

10. Sublinguale Tablette nach einem der Ansprüche 1 bis 9, welche eine Auflösezeit von weniger als 15 Minuten aufweist.

11. Sublinguale Tablette nach einem der Ansprüche 1 bis 10, wobei die Tablette weiter ein oder mehrere Gleitmittel in einer Menge von weniger als 1 Gewichts-% in Bezug auf ein Gewicht der Tablette umfasst, bevorzugt zwischen 0,125 und 0,75 Gewichts-% in Bezug auf ein Gewicht der Tablette, stärker bevorzugt etwa 0,25 Gewichts-% in Bezug auf ein Gewicht der Tablette.

12. Sublinguale Tablette nach einem der Ansprüche 1 bis 11, wobei eine Wirkstoffmenge weniger als 5 mg/Tablette beträgt.

13. Verfahren zur Herstellung einer sublingualen Tablette nach einem der Ansprüche 1 bis 12, umfassend mindestens die folgenden Schritte:
(1) Herstellung von Mikrogranulaten, umfassend ein opioides Analgetikum, geeignet zur sublingualen Verabreichung, durch Sprühen einer Lösung, Suspension oder kolloidalen Dispersion, welche das Opioid, geeignet zur sublingualen Verabreichung, auf neutrale Kerne umfasst; und
(2) Pressen der Mikrogranulate, welche in Schritte 1 erhalten wurden, um die sublinguale Tablette zu erhalten.

14. Verfahren nach Anspruch 13, wobei die Lösung, Suspension oder kolloidale Dispersion, welche das opioide Analgetikum, geeignet zur sublingualen Verabreichung, umfasst, frei von Hilfsstoff ist.

15. Verfahren nach Anspruch 13 oder 14, wobei die Lösung, Suspension oder kolloidale Dispersion, welche das opioide Analgetikum umfasst, weiter mindestens einen pharmazeutisch annehmbaren Hilfsstoff umfasst.

16. Verfahren nach einem der Ansprüche 13 bis 15, wobei das opioide Analgetikum ausgewählt wird aus der Gruppe bestehend aus Buprenorphin, nor-Buprenorphin, Fentanyl, Methadon, Levorphanol, Morphin, Hydromorphon, Oxymorphon, Codein, Oxycodon, Hydrocodon und deren pharmazeutisch annehmbaren Salzen, in einer polymorphen Form, in racemischen oder enantiomeren Form.

17. Verfahren nach einem der Ansprüche 13 bis 16, wobei das opioide Analgetikum ausgewählt wird aus der Gruppe bestehend aus Fentanylbase, Fentanylcitrat, Alfentanyl, Alfentanylhydrochlorid, Sufentanyl, Sufentanilcitrat, Remifentanil, Remifentanilhydrochlorid.

18. Verfahren nach Anspruch 17, wobei Schritt 1 des Verfahrens einen Schritt des Aufbringens einer alkalischen Schicht umfasst.

19. Verfahren nach einem der Ansprüche 13 bis 18, wobei Schritt 1 des Verfahrens einen Schritt des Aufbringens einer pH-modifizierenden Schicht umfasst.

20. Sublinguale Tablette nach einem der Ansprüche 1 bis 12, zur Verwendung in Behandlung von Schmerz, wie Durchbruchschmerz, insbesondere Durchbruchschmerz bei Krebs.

21. Sublinguale Tablette nach Anspruch 20, zur Verwendung in Behandlung von Schmerz in einem Patienten, der bereits unter Opioidtherapie ist.

22. Verwendung der Tablette nach einem der Ansprüche 1 bis 12 zur Herstellung eines Arzneimittels zur Behandlung von Schmerz durch sublinguale Verabreichung der Tablette.

23. Verwendung nach Anspruch 22, wobei der Schmerz Durchbruchschmerz ist, insbesondere Durchbruchschmerz bei Krebs.

24. Verwendung nach Anspruch 23, zur Behandlung von Schmerz in einem Patienten, der bereits unter Opioidtherapie ist.

## Revendications

1. Comprimé sublingual comprenant moins de 20 mg/comprimé d'un analgésique opioïde adapté pour une administration sublinguale, comprenant un diluant directement compressible sous forme de noyaux neutres, dans lequel lesdits noyaux neutres sont essentiellement des granules sphériques comprenant du saccharose et de l'amidon, et dans lequel lesdits noyaux neutres sont revêtus d'au moins une couche active comprenant moins de 5 % en poids du noyau neutre dudit analgésique opioïde adapté pour une administration sublinguale.

2. Comprimé sublingual selon la revendication 1, dans lequel l'analgésique opioïde est un élément choisi dans le groupe consistant en la buprénorphine, la norbuprénorphine, le fentanyl, la méthadone, le lévorphanol, la morphine, l'hydromorphone, l'oxymorphone codéine, l'oxycodone, l'hydrocodone et leurs sels pharmaceutiquement acceptables, sous toute forme polymorphique, sous forme racémique ou énantiomérique.

3. Comprimé sublingual selon la revendication 1, dans lequel l'analgésique opioïde est un élément choisi dans le groupe consistant en une base de fentanyl, le citrate de fentanyl, l'alfentanyl, le chlorhydrate d'alfentanyle, le sufentanil, le citrate de sufentanil, le rémifentanil et le chlorhydrate de rémifentanil.

4. Comprimé sublingual selon l'une quelconque des revendications 1 à 3, dans lequel la couche active est dépourvue de tout excipient.

5. Comprimé sublingual selon l'une quelconque des revendications 1 à 4, dans lequel les noyaux neutres sont revêtus d'une couche de modification du pH.

6. Comprimé sublingual selon la revendication 3, dans lequel les noyaux neutres sont revêtus d'une couche alcaline.

7. Comprimé sublingual selon l'une quelconque des revendications 1 à 6, dans lequel une taille des noyaux neutres est de 100 à 2 000 µm, de préférence de 200 à 600 µm, de manière davantage préférée de 200 à 400 µm.

8. Comprimé sublingual selon l'une quelconque des revendications 1 à 7, ayant une dureté entre 0 et 200 N.

9. Comprimé sublingual selon l'une quelconque des revendications 1 à 8, ayant une friabilité entre 0 et 1 %.

10. Comprimé sublingual selon l'une quelconque des revendications 1 à 9, ayant un temps de désintégration de moins de 15 minutes.

11. Comprimé sublingual selon l'une quelconque des revendications 1 à 10, dans lequel le comprimé comprend en outre un ou plusieurs lubrifiants dans une quantité de moins de 1 % en poids par rapport à un poids du comprimé, de préférence entre 0,125 et 0,75 % en poids par rapport à un poids du comprimé, de manière davantage préférée d'environ 0,25 % en poids par rapport à un poids du comprimé.

12. Comprimé sublingual selon l'une quelconque des revendications 1 à 11, dans lequel une quantité d'ingrédient actif est inférieure à 5 mg/comprimé.

13. Procédé de préparation d'un comprimé sublingual selon l'une quelconque des revendications 1 à 12 comprenant au moins les étapes suivantes :
(1) préparation de microgranules comprenant un analgésique opioïde adapté pour une administration sublinguale par pulvérisation d'une solution, suspension ou dispersion colloïdale comprenant ledit opioïde adapté pour une administration sublinguale sur des noyaux neutres ; et
(2) compression des microgranules obtenus à l'étape 1 de façon à obtenir le comprimé sublingual.

14. Procédé selon la revendication 13, dans lequel la solution, suspension ou dispersion colloïdale comprenant ledit analgésique opioïde adapté pour une administration sublinguale est dépourvue de tout excipient.

15. Procédé selon la revendication 13 ou 14, dans lequel la solution, suspension ou dispersion colloïdale comprenant l'analgésique opioïde comprend en outre au moins un excipient pharmaceutiquement acceptable.

16. Procédé selon l'une quelconque des revendications 13 à 15, dans lequel l'analgésique opioïde est choisi dans le groupe consistant en la buprénorphine, la norbuprénorphine, le fentanyl, la méthadone, le lévorphanol, la morphine, l'hydromorphone, l'oxymorphone codéine, l'oxycodone, l'hydrocodone et leurs sels pharmaceutiquement acceptables, sous toute forme polymorphique, sous forme racémique ou énantiomérique.

17. Procédé selon l'une quelconque des revendications 13 à 16, dans lequel l'analgésique opioïde est choisi dans le groupe consistant en une base de fentanyl, le citrate de fentanyl, l'alfentanyl, le chlorhydrate d'alfentanyle, le sufentanil, le citrate de sufentanil, le rémifentanil et le chlorhydrate de rémifentanil.

18. Procédé selon la revendication 17, dans lequel l'étape 1 du procédé comprend une étape d'application d'une couche alcaline.

19. Procédé selon l'une quelconque des revendications 13 à 18, dans lequel l'étape 1 du procédé comprend une étape d'application d'une couche de modification du pH.

20. Comprimé sublingual selon l'une quelconque des revendications 1 à 12, à utiliser dans le traitement de la douleur telle qu'une douleur paroxystique, en particulier de la douleur paroxystique de cancer.

21. Comprimé sublingual selon la revendication 20, à utiliser dans le traitement de la douleur chez un patient qui est déjà sous thérapie aux opioïdes.

22. Utilisation du comprimé selon l'une quelconque des revendications 1 à 12 dans la fabrication d'un médicament destiné au traitement de la douleur par administration sublinguale dudit comprimé.

23. Utilisation selon la revendication 22, dans laquelle ladite douleur est une douleur paroxystique, en particulier une douleur paroxystique de cancer.

24. Utilisation selon la revendication 23, pour le traitement de la douleur chez un patient qui est déjà sous thérapie aux opioïdes.
